Europäisches Patentamt

European Patent Office

Office européen des brevets

(11) Publication number : **0 485 300 A2**

(12) # EUROPEAN PATENT APPLICATION

(21) Application number : **91403017.6**

(22) Date of filing : **08.11.91**

(51) Int. Cl.⁵ : **G06F 15/42**

(30) Priority : **09.11.90 CU 16990**
**09.11.90 CU 17090**

(43) Date of publication of application :
**13.05.92 Bulletin 92/20**

(84) Designated Contracting States :
**AT BE CH DE DK ES FR GB GR IT LI LU NL SE**

(71) Applicant : **CENTRO DE NEUROCIENCIAS DE CUBA**
**Calle 25 No. 15202, esq. 158, Cubanacan, Playa**
**La Habana 12100 (CU)**

(72) Inventor : **Valdes Sosa, Pedro**
**Calle 224, Edificio No. 2**
**La Coronela, La Lisa (CU)**

Inventor : **Biscay Lirio, Rolando**
**Calle 34 No. 304**
**Miramar, Playa, La Habana (CU)**
Inventor : **Pascual Marqui, Roberto**
**Calle 26 No. 29**
**Vedado, La Habana (CU)**
Inventor : **Neira Blaquier, Livia**
**Taja No. 774**
**Lawton, La Habana (CU)**
Inventor : **Galan Garcia, Lidice**
**Ave 57 No. 10613**
**Mariano, La Habana (CU)**
Inventor : **Jimenez Sobrino, Juan C.**
**Calle 32 No. 62, Cojimar**
**La Habana (CU)**
Inventor : **Bosch Bayard, Jorge**
**Calle 182 Edificio No. 131, Apto. 15**
**Playa, La Habana (CU)**

(74) Representative : **Joly, Jean-Jacques et al**
**CABINET BEAU DE LOMENIE 55, rue d'Amsterdam**
**F-75008 Paris (FR)**

(54) **Method and system for the evaluation and visual display of abnormal electromagnetic physiological activity of the brain andthe heart.**

(57) A method and system for the evaluation and visual display of abnormal electromagnetic physiological activity of the brain and the heart includes a set of bioelectromagnetic amplifiers and a computer based system for the recording and analog to digital conversion of human electromagnetic-physiological signals. The signals collected are expanded in selected spatio-temporal functional bases, and parametric models are used for summarizing the statistical properties of the coefficients. Multivariate topographic maps and curves are computed based on the comparison of the estimated parameters for a patient and the age regression equations for the parameters calculated from a normative database. The multivariate maps and curves are visually displayed in a diagnostically useful color-coded probability scale.

FIG.1

Electrodes or Sensors (1)

↓

Preamplifiers (2)

↓

A/D Converter (3)

↓

Expansion in Functional Basis (4)

↓

Extraction of Parameters (5)

Normative Database (7)

Multivariate Maps and Curves (6)

Display (8)

EP 0 485 300 A2

The present invention relates to electronic computerized medical instruments and more particularly to the analysis and display of bioelectromagnetic signals of the brain and the heart for detecting abnormal functioning by a non-invasive computerized system and method.

Classical electroencephalography and electrocardiography are techniques for the diagnosis of of brain and heart dysfunctions that have been demonstrated to be useful for a number of decades. These methods are largely based on the skill, memory, and experience of a neurologist or cardiologist that examines tracings of bioelectric activity recorded upon paper. Recently, biomagnetic measurements of both the heart and brain have been introduced for the same purposes. Thus, a patient may be examined with any of the following techniques that yield information about current source generators in the human body: electroencephalogram (EEG), magnetoencephalogram (MEG), brain evoked response (ER), electrocardiogram (EKG), and magnetocardiogram (MKG).

In recent years computerized techniques for recording, analyzing and visually displaying electromagnetic signals of the brain and the heart have been introduced, in an attempt to achieve greater objectivity in diagnostic procedures as well as uncovering subtle dysfunctions otherwise difficult to detect except by a highly trained specialist.

The current state of the art, as reflected in US patents 4,417,592; 4,421,112; 4,913,160; and 4,974,598 is as follows:

a) Selected features of the electromagnetic signals of a patient are extracted by means of different data transforms, amongst which the most prominent are: no transform (working with the raw data), broad band spectral analysis, and the Karhunen Loeve expansions.

b) These features are subjected to statistical comparison with standards extracted from a database of normal subjects.

c) As a final result, topographic maps are displayed of the head and torso (for brain and heart data, respectively) in which the intensity of deviation from the norm are color-coded in terms of univariate statistical distributions.

There is abundant evidence (Valdes et al., QEEG in a public health system, Invited lecture at the 2d Congress of Brain Electromagnetic Topography, Brain Topography, 1991, in press) that these procedures are clinically useful in both specialized medical care as well as in the implementation of public health facilities. In spite of these promising results, the sensitivity and specificity of these procedures have been limited to date by a number of factors that include:

i) The feature sets defined to date are too large, presenting multiple multivariate statistical colinearities. Thus, problems arise as to the definition of probability scales and the decision as to whether a deviation in a probability map is actually abnormal. Duffy et al. (Quantified Neurophysiology with Mapping: Statistical Inference, Exploratory and Confirmatory; Data Analysis, Brain Topography Vol. 3 No. 1, Fall 1990 pp. 3-12) have demonstrated the excessively high false positive rates created by this situation.

ii) The data transforms used to date, while useful in some situations, have been demonstrated (Valdes et al.,High Resolution Spectral EEG Norms for Topography, Brain Topography Vol. 3 No. 1, Fall 1990 pp. 281-282) to be too coarse to reconstruct the profile of many abnormalities.

iii) Topographic maps and curves of features are constructed without taking into consideration the high correlation of features due to biological constraints. In fact, abnormalities exists which are essentially multivariate nature.

iv) Topographic maps and curves representing a vector of features are currently color-coded only using the marginal (univariate) probability distribution of each component of the vector. This increases the probability of false positive findings when searching for significant regions in the whole map or curve.

The objective of the present invention is a method and system for the evaluation and visual display of abnormal electromagnetic physiological activity of the brain and heart for the calculation of topographic maps and curves with increased sensitivity and specificity.

In accordance with a first aspect of the present invention there is provided a method for evaluating and displaying the abnormal electromagnetic physiological activity of a patient, said method including the steps of:

a) Attaching or approximating a set of electrodes and magnetic sensors to the scalp or torso of a patient to detect electromagnetic physiological activity in the form of electroencephalogram (EEG), magnetoencephalogram (MEG), brain evoked response (ER), electrocardiogram (EKG), and magnetocardiogram (MKG);

b) Amplifying the said electromagnetic signals detected at each electrode and magnetic sensor;

c) Obtaining on-line digital spatio-temporal signals, consisting of said EEG, MEG, ER, EKG, and MKG, by connecting analog-digital converters to each amplifier, and digitizing all data as it is gathered;

d) Obtaining sets of said spatio-temporal signals for the patient, and computing the coefficients of its expansions in a spatio-temporal functional basis defined by the tensor product of selected functional bases for the spatial and temporal domains;

e) Computing estimates of the parameters determined by statistical parametric models for the probabilistic moments of said coefficients for a patient;

f) Automatic construction of summarized topographic maps by a process of diagnostically useful summarization of the information contained in said sets of signals or probabilistic moments corresponding to different instants or frequencies, and automatic construction of summarized curves by a process of summarization of the information contained in said sets of signals or probabilistic moments corresponding to different electrodes and magnetic sensors;

g) Computing multivariate topographic maps and curves based on the comparison of said summarized topographic maps and curves with their means, standard deviations and correlations in stored normative data bases from normal subjects;

h) Visually displaying said multivariate topographic maps and curves in a diagnostically useful color-coded probability scale.

In accordance with a second aspect of the invention there is provided a system for evaluating and displaying the abnormal electromagnetic physiological activity of a patient, said system including:

a) A set of electrodes and/or magnetic sensors adapted to be attached or approximated to the scalp or torso of a patient in order to detect electromagnetic physiological activity in the form of electroencephalogram (EEG), magnetoencephalogram (MEG), brain evoked response (ER), electrocardiogram (EKG), and magnetocardiogram (MKG);

b) A system for the amplification of the aforementioned physiological activity;

c) Means for the analog-to-digital conversion of the preamplified physiological activity in the form of digital spatio-temporal signals, representative of the aforementioned EEG, MEG, ER, EKG, and MKG;

d) Means for storing sets of said spatio-temporal signals for the patient, and for computing the coefficients of its expansions in a spatio-temporal functional basis defined by the tensor product of selected functional bases for the spatial and temporal domains;

e) Means for computing estimates of the parameters determined by statistical parametric models for the moments of said coefficients for a patient;

f) Means for the automatic construction of summarized topographic maps by a process of diagnostically useful summarization of the information contained in said sets of signals or probabilistic moments corresponding to different instants or frequencies, and automatic construction of summarized curves by a process of summarization of the information contained in said sets of signals or probabilistic moments corresponding to different electrodes and magnetic sensor;

g) Means for computing multivariate topographic maps and curves for a given patient based on the comparison of said summarized topographic maps and curves with their means, standard deviations and correlations in stored normative data bases from normal subjects;

h) Means for visually displaying said multivariate topographic maps and curves in a diagnostically useful color-coded probability scale.

In order that the invention may be better understood, further detailed description follows with reference to the accompanying drawings in which:

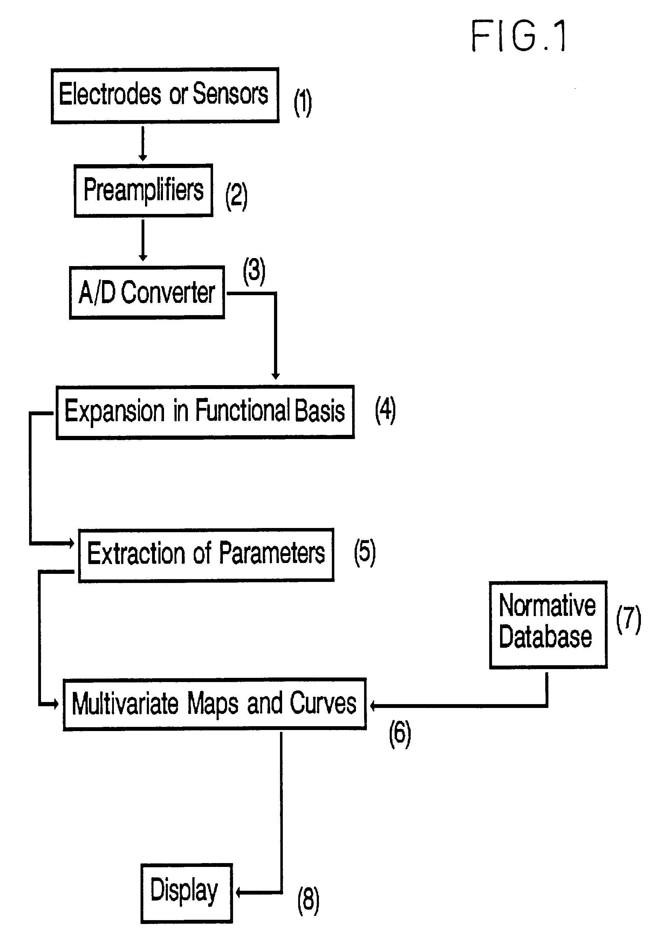

Figure 1) is a block schematic drawing of one embodiment of the present invention.

Figure 2) shows the regression surfaces determined by the development of the EEG spectrum at each electrode as function of the age of the subject in the normal population.

Figure 3) shows the narrow band Z-spectrum at derivation P4 of a patient with a right parietal meningioma as compared to the Broad Band Spectral representation of the same patient.

Figure 4) shows the Z map corresponding to the maximal deviation of Figure 3 maximum probability scale for a patient with a central cyst detected by Nuclear magnetic resonance images.

Figure 5) shows a Topographic Brain Mahalanobis Map with the maximum probability scale for a patient with a deep frontotemporal tumor.

Figure 6) shows a Topographic Brain Map of conditional Z values with the maximum probability scale for the same patient as in figure 5.

The detection of the patient's physiological activity may be made using a plurality of conventional detectors (electrodes) for electrical activity and magnetometers or gradiometers for the magnetic activity. The sensors and detectors (1) will be placed according to a pre-defined plan in order to maximize the amount of information to be extracted from the bioelectromagnetic signals, in particular the sensor (detector) array will be placed in proximity upon the head (torso) for the study of brain (heart) activity.

The electric and/or magnetic (termed henceforth electromagnetic) signals are amplified (2) to the dynamic range of an Analog to Digital converter (3), which converts the signals into numbers that are stored in the memory of a general purpose digital computer.

Let $Y(x_m,t_n)$ denote the electromagnetic signal recorded at electrode position $x_m$ at sensor position $X_m$ at time $t_n$ ($t_1,...,t_n$ are the times of digitization). Information is recorded in buffers of size n called segments and which are the complete set of samples for M sensor/detectors and a time period of size $T = n\Delta t$, where $\Delta t$ is the sampling period.

Segments are projected upon the tensor product of a spatial basis $\{A_1(x), A_2(x),...\}$ and a temporal basis, $\{B_1(t), B_2(t),...\}$, by least squares fitting of the expansion:

$$Y(x,t) = \Sigma\Sigma C_{ij}A_i(x)B_j(t) \quad (1)$$

in terms of which the data $Y(x_m,t_n)$ is now-represented by the coefficients $C_{ij}$ (4). This representation has advantages in terms of data compression, statistical orthogonalization and interpretation, though the specific basis, and the number of its elements, must be selected according to the type of data collected by the user.

Important temporal bases to be used are:

a) The canonical vector basis in $R^n$, which reduces to using the raw data for analyzing instantanous temporal phenomena.

b) The Fourier basis $B_j(t) = \exp(i\omega_j t)$ which transforms the data into the frequency domain for the analysis of stationary stochastic phenomena.

c) The Karhunen-Loeve basis, eigenfunctions of the second order moments of the signal, either in the time or frequency domain, also known as Principal Components for the analysis of general stochastic phenomena.

d) The wavelet basis on the real line, of the form:

$B_j(t) = g((t-a_{j1})/b_{j2})$ or $B_j(t) = g((t-a_{j1})\exp(i\omega_{j2})$, where g is a convenient waveform or "mother wavelet" (e.g. a Gaussian), $j= (j_1,j_2)$, $a_{j1}$ is interpreted as a translation parameter, $b_{j2}$ as a scale parameter and $\omega_{j2}$ as a modulation (frequency) parameter. This type of basis is convenient for time localization of nonstationary stochastic phenomena and multiresolution analysis.

Important temporal bases to be used are:

a) The canonical vector basis in $R^m$, which reduces to using the raw data for analyzing instantanous spatial phenomena.

b) The Karhunen-Loeve basis, eigenfunctions of the second order moments of the signal, in the space domain.

c) Spatial spline basis of the general form:

$$B_j(x) = \Sigma \varphi_k(x)\varphi_k^*(x_j)/\lambda_k$$

where $\varphi_k$ and $\lambda_m$ are, respectively, the eigenfunctions and eigenvalue of an operator D that acts upon functions defined over the surface S on which the sensors/detectors are placed or approximated to $x_j$. This basis has the property that any function f on S is interpolating the points $x_j$ with arbitrary values $y_j$ under the restriction of being the smoothest in the sense of minimizing $||Df||$. In particular for S a sphere, (approximation to the head or torso)

$$B_j(x) = \Sigma \Sigma Y_{lm}(x)Y^*_{lm}(x_j)/[l(l + 1)]^2$$

where $Y_{lm}$ are the spherical harmonic functions.

The statistical properties of the coefficients of the spatio-temporal signals are summarized by their statistical moments. For reasons of simplicity, only up to second order moments will be considered (all the information necessary for gaussian processes):

$$\mu_{ij}= E(C_{ij})$$

$$\mu_{ijkl}=E(C_{ij}C_{kl}^*)$$

where E() is the expectation operator and * is transpose conjugation operator.

These moments are described in terms of a minimal set of parameters:

$$\mu ij=\mu_{ij}(\theta)$$

$$\mu_{ijkl}=\mu_{ijkl}(\theta)$$

where the $\mu$ (.) are known functions. This parametric description reduces the size of final parameter set based upon the smoothness of variation of the statistical moments in the spatial and temporal domains.

Important parametric models to be used for the statistical moments of the coefficients are:

a) The model defined by taking the moments as parameters, so that no functional structure is introduced. A remarkable example is the case where the vector of parameters is the discrete spectrum at each electrode or sensor (i.e. the second order moments of the coefficients associated with the Fourier expansion for the time domain).

2) The model obtained by considering the vector of coefficients $C(t) = (C_j(t))$ of a spatial expansion

$$Y(x,t) = \Sigma \Sigma C_{ij}A_i(x)B_j(t) = \Sigma C j (t) B j(x)$$

as a multivariate autoregressive time series. Thus, the second order statistical moments are functions of the autoregressive parameters.

3) Source models, such as a multiple dipole source model, with or without varying locations and moments.

In this type of model the statistical moments of the coefficients are parameterized by taking the statistical moments of the source as parameters.

In general, the specific parametric model must be selected according to the type of data collected by the user.

The extraction of parameters (5) for a given patient consists of its estimation by optimizing a statistical distance (least squares or minimum likelihood metric) between the parameterized statistical moments and the sample moments (averaging across all the time segments recorded).

The trends and variabilities of the parameters V (may be transformed in order to achieve a Gaussian distribution) are analyzed by means of regression equations:

$$V = g(u;\beta) + e$$

where $e$ is a random vector with zero mean and vector of variances $h(u;\gamma)$; $g(u;\beta)$ is the mean regression function; and $\beta$ and $\gamma$ are the vectors of unknown coefficients in the expansions of $g$ and $h$ in given functional bases respectively. The functions $g$ and $h$ allow to take into account the smoothness of the the variation of V as a function of age and other covariables included in $u$.

These regression equations are fitted (using least squares regression analysis) to the data corresponding to a large sample of normal subjects. A Normative Database (7) stores all the information about the trends and variabilities associated with the aforementioned regression equations in the normal population, which includes stimates of: i) the mean regression function $g$ (i.e., the estimation of $\beta$), and also the variances and correlations, ii) the vector of residuals of the parameters, $V-g(u;\hat{\beta})$, iii) the vector of residuals of the moments of the coefficients, $m - m(\hat{\theta})$, and iv) the estimate of the vector of residuals the original signals, $Y(x,t)-\hat{Y}(t,x)$. Here $\hat{Y}(x,t)$ denotes the predited value of Y:

$$\hat{Y}(x,t) = \Sigma \Sigma \hat{C}_{ij} A_i(x) B_j(t)$$

where $\hat{C}_{ij}$ is the predicted value of $C_{ij}$ using the estimated first order moment $\mu_{ij}(\hat{\theta})$.

Given a patient's data, the information stored in the normative database (7) is used to compute multivariate topographic maps and curves (6), the symbols M(x) and Q(v) denoting a patient's multivariate map and curve respectively. M(x) is any spatial vector of features, Q(x) is a temporal or frequency vector of features.

In what follows Z will generically denote a map or a curve and $\mu$ y $\Sigma$ shall refer to it's mean vector and dispersion matrix in the normal population. From Z the following multivariate maps and curves will be calculated:

a) Typified conditional residual Maps or curves defined by $R = (Z-\hat{Z})$, where $\hat{Z}$ is the vector with components $\hat{Z}_{.j} = E(Z_j/Z^{(j)})$, the conditional expected value of $Z_j$ given the rest of the components of Z. This vector is scaled in terms of its standard deviation.

b) Typified Maximal Abnormality Direction Map or Curve defined by $R = \Sigma^{-1} Z$ which is the direction which maximizes $(R'Z)^2 /(R'\Sigma R)$. This vector is also scaled in terms of its standard deviation.

The multivariate Map or Curve is visually displayed as a color coded map in which the intensity value $I_j$ of a given pixel is scaled according to:

$$I_j = \begin{cases} 1 - F(R_j), & \text{when high values of } R_j \text{ are abnormal} \\ G(R_j), & \text{when low values of } R_j \text{ are abnormal} \end{cases}$$

where F() and G() are, respectively, the probability distribution function of the maximum and the minimum of the vector R.

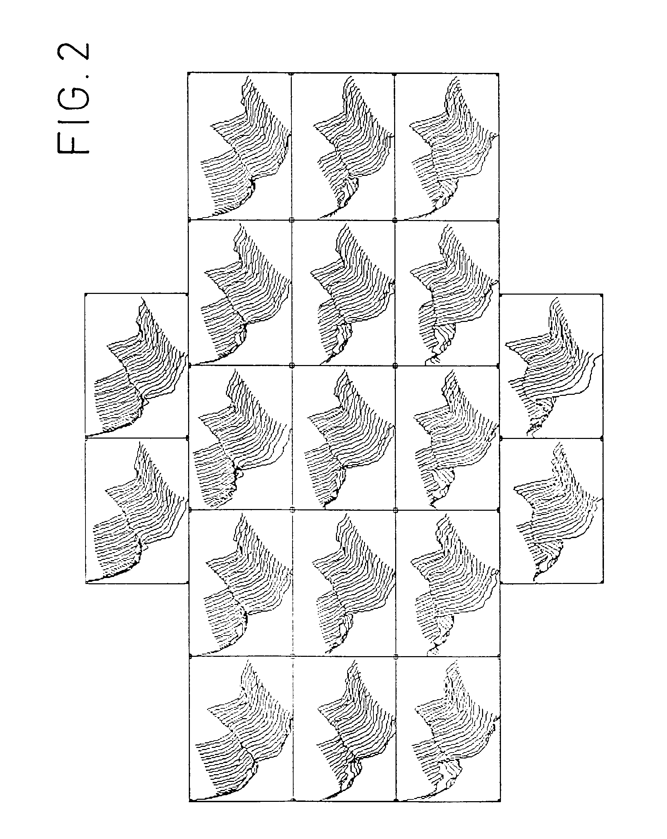

Examples of the use of these procedures in clinical material will now be given. If one uses as the spatial basis the canonical one, as a temporal basis the Fourier basis, a high resolution frequency decomposition of the EEG may be obtained. The parameters of interest are the variances of the Fourier coefficients (spectra) at each derivation. The age dependent regression equations for the normal population are expressed as tensor products of a frequency spline basis and polynomial functions for age dependency. Figure 2 illustrates the frequency-age mean value for the log spectra in all derivations of the 10/20 system. The multivariate map and curve for a given derivation are termed in this case the high resolution "z map" and "z spectrum", respectively.

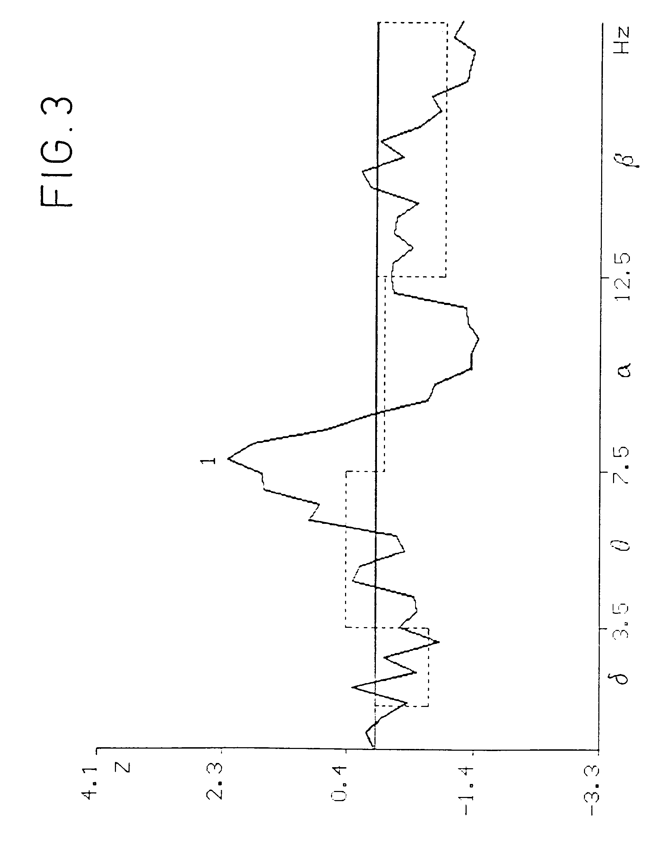

Figure 3 illustrates the higher sensitivity of the high resolution Z-spectrum at derivation P4 of a patient with a right parietal meningioma (dark curve) as compared to traditional Broad Band Spectral representation (light curve) of the same patient (Figure 3).

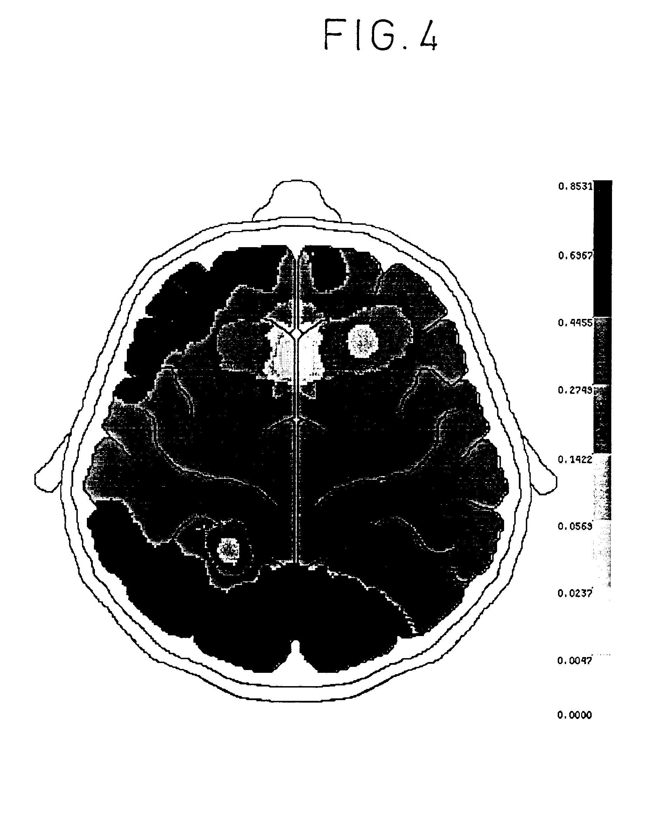

At the maximum of abnormality evidenced in Figure 3, a multivariate Z map (Figure 4) coded with a maximum probability scale is maximal at the same location as that of a central cyst detected by nuclear magnetic resonance images.

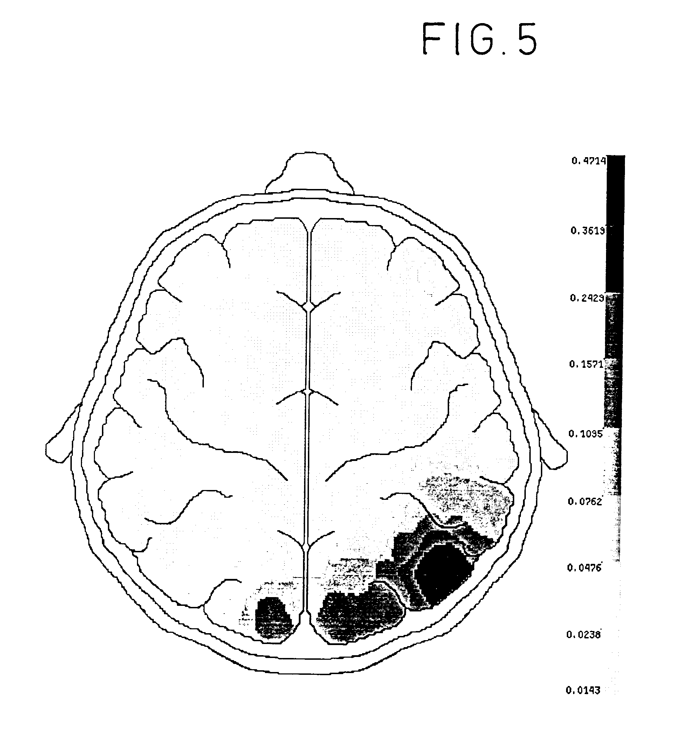

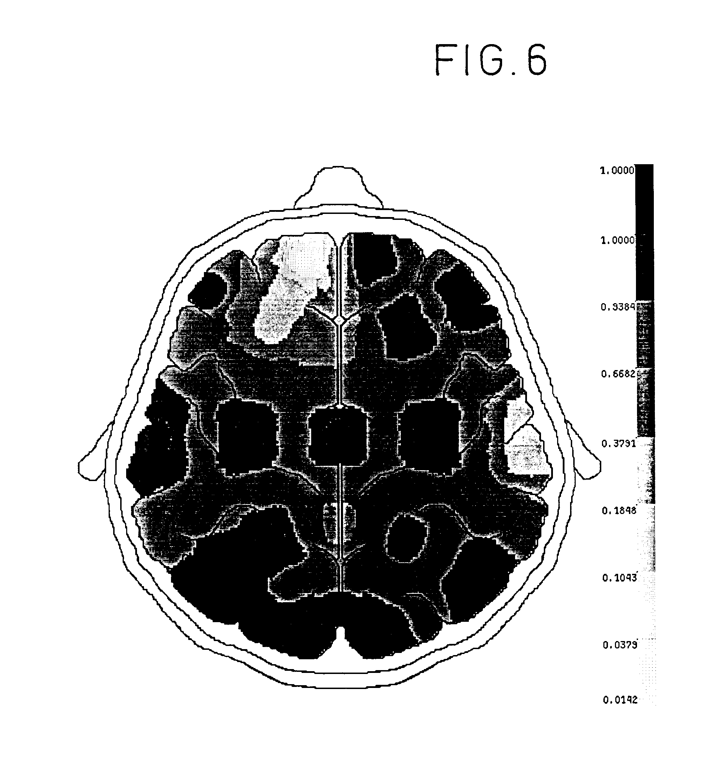

The use of Multivariate maps is further illustrated in Figures 5 and 6. Figure 5 shows a Topographic Brain Mahalanobis Map with the maximum probability scale for a patient with a deep left frontotemporal tumor. Figure 6 shows a Topographic Brain Map of conditional Z values with the maximum probability scale for the same patient as in figure 5.

## Claims

1. 1. A method for the evaluation and visual display of abnormal electromagnetic physiological data of the brain and the heart, said method including the steps of:

   a) Attaching or approximating a set of electrodes and magnetic sensors to the scalp or torso of a patient to detect electromagnetic physiological activity in the form of electroencephalogram (EEG), magnetoencephalogram (MEG), brain evoked response (ER), electrocardiogram (EKG), and magnetocardiogram (MKG);

   b) Amplifying the said electromagnetic signals detected at each electrode and magnetic sensor;

   c) Obtaining on-line digital spatio-temporal signals, consisting of said EEG, MEG, ER, EKG, and MKG, by connecting analog-digital converters to each amplifier, and digitizing all data as it is gathered;

   d) Obtaining sets of said spatio-temporal signals for the patient, and computing the coefficients of its expansions in a spatio-temporal functional basis defined by the tensorial product of selected functional bases for the spatial and temporal domains, ;

   e) Computing estimates of the parameters determined by statistical parametric models for the probabilistic moments of said coefficients for a patient;

   f) Automatic construction of summarized topographic maps by a process of diagnostically useful summarization of the information contained in said sets of signals or probabilistic moments corresponding to different instants or frequencies, and automatic construction of summarized curves by a process of summarization of the information contained in said sets of signals or probabilistic moments corresponding to different electrodes and magnetic sensors;

   g) Computing multivariate topographic maps and curves based on the comparison of said summarized topographic maps and curves with their means, standard deviations and correlations in stored normative data bases from normal subjects;

   h) Visually displaying said multivariate topographic maps and curves in a diagnostically useful color-coded probability scale.

2. A method as claimed in claim 1 wherein the said functional basis for the temporal domain is the trigonometric basis (i.e., the Fourier expansion); said parameters are the variances of the Fourier coefficients (i.e., the spectrum) for each electrode and frequency, which are estimated by the sample spectrum or periodogram; and normative mean values, standard deviations and correlations of the periodogram are obtained by non-linear and heteroscedastic regression analysis of the log-periodogram as function of frequency and age for each electrode, using a stored normative data base of normal subjects; said regression analysis being based upon mean and variance regression functions constructed by the tensorial product of a polynomial basis for the age and a spline basis for the frequency domain.

3. A method as claimed in claim 1 wherein said functional basis is constructed by the tensorial product of the trigonometric basis (i.e. Fourier expansion) for the time domain and the reproducing kernel basis determined by the positions of the set of electrodes and a norm of functional smoothness for the spatial domain; and said parameters are the parameters of a multivariate autoregressive time series model for the vector of coefficients of the expansion in said spatial functional basis.

4. A method as claimed in claim 1 wherein said parametric model for said probabilistic moments is based upon a given source model, such as a multiple dipole source model, with or without time varying locations and moments.

5. A method as claimed in claim 1 wherein the temporal and/or spatial basis are wavelet bases in order to account for nonstationarities and for multiresolution representation of said sets of signals.

6. A method as claimed in claim 1 wherein the multivariate topographic maps and curves of a given patient are obtained by computing the residual of each value of the patient's summarized map or curve with respect to its expected value, given the remaining values of the patient, in accordance to the probability distribution of the normative population stored in a computer memory.

7. A method as claimed in claim 1 wherein the multivariate topographic maps and curves of a given patient are obtained by computing the vector which points in the direction of maximal statistical deviation of the patient's summarized map or curve from the map or curve representative of the normative population.

8. A method as claimed in claim 1 wherein the visual display of said multivariate topographic maps and curves is carried out using the probability values defined by the distribution functions of the maximum and minimum of said maps and curves stored in a normative database, and the representation of these probability values by a color-coded scale.

9. A system for the evaluation and visual display of abnormal electromagnetic physiological data of the brain and the heart that comprises:

a) A set of electrodes and/or magnetic sensors adapted to be attached or approximated to the scalp or torso of a patient in order to detect electromagnetic physiological activity in the form of electroencephalogram (EEG), magnetoencephalogram (MEG), brain evoked response (ER), electrocardiogram (EKG), and magnetocardiogram (MKG);

b) A system for the amplification of the aforementioned physiological activity;

c) Means for the analog-to-digital conversion of the preamplified physiological activity in the form of digital spatio-temporal signals, representative of the aforementioned EEG, MEG, ER, EKG, and MKG;

d) Means for storing sets of said spatio-temporal signals for the patient, and for computing the coefficients of its expansions in a spatio-temporal functional basis defined by the tensorial product of selected functional bases for the spatial and temporal domains;

e) Means for computing estimates of the parameters determined by statistical parametric models for the moments of said coefficients for a patient;

f) Means for the automatic construction of summarized topographic maps by a process of diagnostically useful summarization of the information contained in said sets of signals or probabilistic moments corresponding to different instants or frequencies, and automatic construction of summarized curves by a process of summarization of the information contained in said sets of signals or probabilistic moments corresponding to different electrodes and magnetic sensors;

g) Means for computing multivariate topographic maps and curves for a given patient based on the comparison of said summarized topographic maps and curves with their means, standard deviations and correlations in stored normative data bases from normal subjects;

h) Means for visually displaying said multivariate topographic maps and curves in a diagnostically useful color-coded probability scale.

FIG.1

```
┌─────────────────────────┐
│  Electrodes or Sensors  │  (1)
└─────────────────────────┘
            │
            ▼
   ┌──────────────────┐
   │  Preamplifiers   │  (2)
   └──────────────────┘
            │
            ▼                (3)
   ┌──────────────────┐
   │  A/D Converter   │
   └──────────────────┘
            │
            ▼
┌──────────────────────────────┐
│ Expansion in Functional Basis │  (4)
└──────────────────────────────┘

   ┌─────────────────────────┐
   │ Extraction of Parameters │  (5)
   └─────────────────────────┘

                                        ┌──────────────┐
                                        │  Normative   │  (7)
                                        │  Database    │
                                        └──────────────┘

┌──────────────────────────────┐
│ Multivariate Maps and Curves  │◄────
└──────────────────────────────┘  (6)
            │
            ▼
   ┌──────────────┐
   │   Display    │  (8)
   └──────────────┘
```

FIG. 2

FIG. 3

# FIG.4

# FIG.5

# FIG.6